# EUROPEAN PATENT APPLICATION

(11) **EP 2 933 245 A1**
(43) Date of publication of application: **21.10.2015**
(21) Application number: 14165039.0
(22) Date of filing: 17.04.2014
(51) Int. Cl.: C07C 269/04, C07C 271/28

(54) **Oxidative carbonylation of amino aryl compounds by aryl formates**

(71) Applicant: Bayer Technology Services GmbH, 51368 Leverkusen (DE)
(72) Inventor: Dr. Lolli, Giulio, 51069 Köln (DE); Dr. Wolf, Aurel, 42489 Wülfrath (DE); Prof. Dr. Mleczko, Leslaw, 41542 Dormagen (DE); Yalfani, Mohammad S., 52074 Aachen (DE)
(74) Representative: BIP Patents

(57) **Abstract**

The field of the present invention relates to a process for preparing alkylaryl carbamates from alkyl formates and at least one amino aryl compound in the presence of catalysts, and to the use thereof for preparation of isocyanates.

## Description

The field of the present invention relates to a process for continuously preparing alkylaryl carbamates and diarylcarbamates from aryl formates and at least one amino aryl compound in the presence of a catalyst, and to the use thereof for preparation of isocyanates (NCO) or polyurethanes (PU).

Carbamate synthesis currently receives particular attention as carbamates provide a safer access compared to the classic phosgene route to isocyanates, the main component for manufacturing of polyurethanes (Kreye et al., Green Chem., 2013, 15, 1431-1455).

The catalytic carbonylation of amines or nitro compounds as described in WO2013012814 A2 and the reaction of amines with dimethyl carbonate, described in EP0065026 A1, are the most promising routes for the synthesis of carbamates (Aresta et al., Tetrahedron, 1991, 47, 9489-9502; Tafesh et al., Chem. Rev., 1996, 96, 2035-2052; Selva et al., J. Org. Chem. 2005, 70, 2771-2777; Distano et al., Appl. Catal. B: Environ., 2006, 66, 72-80; Ragaini et al., Dalton Trans., 2009, 6251-6266).

WO2013012814 A2 describes a method for preparing a carbonate or carbamate, by reacting R₁OH with carbon monoxide (CO) and oxygen adsorbed on a metallic gold catalyst and R₂XH according to the following scheme: 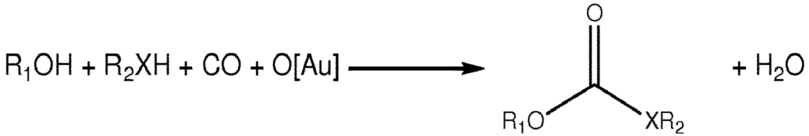 wherein X is O or NR₂*, wherein each of R₁, R₂, and R₂* is independently selected from the group consisting of C₁-C₈ straight chain alkyl, C₃-C₈ branched alkyl, C₃-C₈ cycloalkyl, and C₆-C₁₀ aryl. In the process as described, carbonylation of amines or nitro compounds requires CO, which despite the good reactivity as carbonylating agent, is classified as toxic gas and has selectivity problems.

EP0065026 A1 describe a process for preparing a carbamate from an organic carbonate and an aromatic amine in the presence of catalytic quantity of a Lewis acid catalyst characterized by employing as the Lewis acid catalyst a catalyst which is soluble in the reaction mixture at the reaction conditions employed and which is at least one member selected from the group consisting of a zinc or divalent tin halide, a zinc or divalent tin salt of a monovalent organic compound which has a pKa value of at least 2.8, and a zinc or divalent tin salt of trifluoroacetic acid. The main issue of this method is the requirement of an organic carbonate as starting material. Organic carbonates, like dimethyl carbonate (DMC) are industrially synthesized by reaction of the corresponding alcohols with phosgene, a highly toxic intermediate. Few methods are described to produce phosgene-free dimethyl carbonate: oxidative carbonylation of methanol (MeOH) or ring opening of epoxides followed by Transesterification (Romano et al., Ind. Eng. Chem. Prod. Res. Dev. 1980, 19, 396-403; Bhanage et al., Appl. Catal. A: Gen., 2001, 219, 259-266).

The disadvantages of these routes are low yield and the stoichiometric formation of glycols as by-product, respectively. Therefore, a safe, highly efficient, green and economically viable approach is required for the production of carbamates from amines or nitro precursors.

Formic acid and its derivatives such as methyl formate (MF) have shown wide application as C1 building block in the synthesis of basic chemicals (Lee et al., Appl. Catal. 1990, 57, 1-30; Morimoto et al., Angew. Chem. Int. Ed. 2004, 43, 5580-5588).

Recent studies have focused on carbon recycling by the conversion of carbon dioxide (CO₂) into formic acid and its derivatives (W. Leitner, Angew. Chem. Int. Ed. Engl., 1995, 34, 2207-2221; Markewitz et al., Energy Environ. Sci., 2012, 5, 7281-7305; Peters et al., ChemSusChem, 2011, 4, 1216-1240; Olah et al., J. Org. Chem., 2009, 74, 487-498; Cokoja et al., Angew. Chem. Int. Ed., 2011, 50, 8510-8537; Hölscher et al., Z. Naturforsch, 2012, 67b, 961-975; Quadrelli et al., ChemSusChem, 2011, 4, 1194-1215; Sakakura *et al.,* J.-C. Choi and H. Yasuda, Chem. Rev., 2007, 107, 2365-2387).

This hydrogenation reaction is favorable when it is performed under basic conditions, resulting in formate salt which in the presence of methanol is separable as methyl formate (MF) (Federsel et al., Chem. Eur. J., 2012, 18, 72-75; Federsel et al., Angew. Chem. Int. Ed., 2010, 49, 6254-6257; Federsel et al., Angew. Chem. Int. Ed., 2010, 49, 9777-9780; Kerry et al., J. Am. Chem. Soc., 2007, 129, 6360-6361).

Methyl formate can be activated by a strong base such as alkali methoxide (MOCH₃) at mild reaction temperatures (25-80 °C) which results in its decarbonylation (Carpentier et al., Tetrahedron Lett. 1991, 32, 4705-4708). Methyl formate behaves as weak acid and is deprotonated by strong base and rapidly dissociated to CO and methanol creating an equilibrium (Ramirez-Vega et al., J. Mol. Catal. A: Chem., 1995, 96, 15-20). Such an equilibrated system can be used in the carbonylation reaction along with an appropriate catalyst.

The use of methyl formate in the carbonylation reaction, not only introduces it as safe and green agent, but in case of methyl formate produced by CO₂ hydrogenation, also represents an actual recovering of carbon dioxide into important industrial chemicals. Recently, methyl formate was used in the carbonylation of phenol to methyl phenyl carbonate (Yalfani et al., Green Chem., 2013, 15, 1146-1149).

There is therefore a need for a method for producing alkylaryl carbamates and diarylcarbamates that provides high security standards and employs "green" reactants.

This problem was solved by using alkyl formates as carbonylating agent for amino aryl compounds in presence of at least one catalyst.

Alkyl formates which are suitable in the context of the invention are preferably alkyl formates from C1 to C6. A particularly preferred alkyl formate is methyl formate. Methyl formate (MF) is not only an environmentally friendly, green and efficient carbonylating agent, compared to toxic CO gas, but surprisingly it showed also better performance compared to gas phase CO.

Amino aryl compounds which are suitable in the context of the invention are preferably aniline derivatives of the general formula PhNH₂RR'(I) 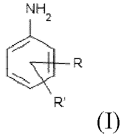 wherein R and R' are each independently selected from the group comprising NH₂, H, alkyl, aryl or aminoaryl groups.

Examples of amino aryl compounds are aniline, aminotoluene, diaminotoluene (TDA) - 2,4-TDA, 2,6-TDA - and methylenedianiline (MDA) - 2,4-MDA, 2,6-MDA, 4,6-MDA, 4,4'-MDA, pure or as a mixture of isomers for the preparation of the corresponding carbamates (2,4-DMTC, 2,6-DMTC, 2,4-DMMC, 2,6-DMMC, 4,6-DMMC, 4,4'-DMMC respectively). Also a mixture of aminoaryl compounds may be used as a reactant leading to a mixture of corresponding carbamates.

Of particular commercial interest are the reactions of 2,4-diaminotoluene (2,4-TDA) and 4,4'-methylenedianiline (4,4'-MDA) with methylformates for the preparation of methylaryl carbamates, such as methyl(2-methylphenyl)carbamate, dimethyltoluene-2,4-dicarbamate (2,4-DMTC) and 4,4'-bis(carbomethoxyamino)diphenylmethane (4,4'-DMMC) respectively, pure or as a mixture of isomers.

Most interesting is the use of 2,4-diaminotoluene (2,4-TDA) for the preparation of dimethyltoluene-2,4-dicarbamate.

The process is also of particular interest for the production of methyl phenylcarbamate (MPC) by oxidative carbonylation of aniline with methyl formate, which subsequently can be decomposed to phenylisocyanate.

Preferably 2,4-diaminotoluene (2,4-TDA) can be converted to dimethyl toluene-2,4-dicarbamate (2,4-DMTC) to further produce toluene 2,4-diisocyanate (TDI) and 4,4'-methylenedianiline (4,4'-MDA) can be converted in 4,4'-bis(carbomethoxyamino)diphenylmethane (4,4'-DMMC) to further produce 4,4'-methylenediphenylene diisocyanate (4,4'-MDI).

The preferred catalysts in the present invention are metal-based redox catalysts, preferably on Palladium basis. Most preferred catalysts are PdBr₂, Pd(OAc)₂, Pd(salen) (salen: *N,N'-*bis(salicylidene)ethylenediamine), PdCl₂(CH₃CN)₂, Pd/C, Pd/SiO₂ and Pd/Al₂O₃. In a preferred embodiment the Palladium concentration of the redox catalyst Pd/C, Pd/SiO₂ or Pd/Al₂O₃ is from 1 wt% to 20 wt%, preferably from 5 wt% to 10 wt%.

In a preferred embodiment a co-catalyst is used to increase the yield of product. A co-catalyst according to the present invention is another species that it is added to further improve the activity of the catalyst. The co-catalyst alone could be a catalyst itself. The combination of the catalyst and co-catalyst results in an overall increase of the reaction rate compared to the use of only one of them. Co-catalysts according to the preferred embodiment are on iodide basis, preferably Nal, KI, LiI and CsI.

In a further preferred embodiment alkyl formate is activated by alkali metal alkoxides MOCH₃, wherein M is an alkali metal. The alkali metal M is preferably Na or K.

Most preferred combination of catalysts and co-catalyst is Pd/C and NaI optionally in the presence of NaOCH₃ as an activator.

In an industrial application the stoichiometry of the reactants is usually approximately 1:1.

The reaction may be conducted from room temperature to 250 °C. In a preferred embodiment the reaction temperature is from 80 °C to 250 °C, preferably from 140 °C to 160 °C.

Similarly although reaction may be conducted at atmospheric pressure it is preferred to use a reaction pressure from 10 bar to 200 bar, preferably from 30 bar to 80 bar for better industrial usability.

It is possible to use the alkylaryl carbamates obtainable from the method according to the present invention for the production of isocyanates, such as 2,4-toluene diisocyanate (2,4-TDI) and 4,4'-methylenebis(phenyl isocyanate) (4,4'-MDI).
**Fig. 1****:** Products distribution during oxidative carbonylation of aniline with methyl formate using the method of the invention at 140 °C.
**Fig. 2****:** Product distribution during oxidative carbonylation of aniline with CO/CH₃OH at 140 °C according to the method of the art.
**Fig. 3****:** Product distribution obtained in the oxidative carbonylation of aniline with methyl formate after 2 h reaction in the temperature range 80-160 °C (Working Example 1).
**Fig.** 4: Pressure profiles of the oxidative carbonylation of aniline using methyl formate according to the method of the invention in comparison to the state of the art making use of CO/CH₃OH at 140 °C in 2 h.

### Examples

Hereinafter, the present invention and the best mode for carrying out the present invention are described in more detail and specifically with reference to the examples, which however are not intended to limit the present invention.

The chemicals used, producer and catalog number is summarized in Table 1.

**Table 1: List of the used chemicals, their suppliers and purities.**

| **Chemical** | **Supplier (CN*)** | **Purity (%)** |
|---|---|---|
| Aniline | Sigma-Aldrich (242284) | 99.5 |
| NaOCH3 solution 25 wt% in methanol | Sigma-Aldrich (156256) | 25 |
| Methyl formate | Sigma-Aldrich (291056) | 99 |
| Pd/C | Sigma-Aldrich (20569-9) | 10 |
| Pd/C | Sigma-Aldrich (205680) | 5 |
| Pd/C | Sigma-Aldrich (205672) | 1 |
| Pd/Al₂O₃ | Sigma-Aldrich (205710) | 5 |
| Pd/SiO₂ | Sigma-Aldrich (AB155784) | 5 |
| Sodium iodide | Sigma-Aldrich (383112) | 99.5 |
| Methyl N-phenyl carbamate | ABCR (AB140601) | 98 |
| Methanol | Sigma-Aldrich (179957) | 99.6 |

### Working Example 1: Oxidative carbonylation of aniline with methyl formate

A 160 mL Parr autoclave pressure reactor was charged with methyl formate (30 mL, 0.49 mol), NaOCH₃ solution 25 wt% in methanol (0.115 ml, 0.5 mmol), supported Pd catalyst (0.011 mmol atom Pd) as listed in Table 2, and NaI (90 mg, 0.6 mmol). The reaction mixture was stirred at 700 rpm and heated to the desired temperature (80-160 °C). After the temperature had stabilized and the internal pressure (Pᵢₙ) had become almost constant, the reactor was pressurized with artificial air (50% of Pᵢₙ). Thereafter, aniline (0.93 g, 10 mmol) dissolved in methanol (5 mL) was injected into the reactor (4 mL/min) using a high pressure HPLC pump. The injection of aniline was considered as start of the reaction (t = 0). After 2h, the reaction was quenched by cooling the reactor with ice-water. The autoclave was opened and the reaction products were analyzed by Gas Chromatography (GC) and Gas Chromatography-Mass Spectrometry (GC-MS).

GC analysis conditions were as follows: Instrument: Thermo SCIENTIFIC TRACE GC Ultra, Column: OPTIMA 5 Amine (30 m × 0.25 mm × 0.4 µm); 80-280 °C, 5 min isothermal, 12 °C/min, 30 min isothermal; FID 250 °C; methyl benzoate as external standard.

GC-MS analysis conditions were as follows: Instrument: VARIAN CP3800 Gas Chromatograph - VARIAN 1200L Quadrupole MS/MS; Column: SE34 (30m × 0.32 mm); 80-280 °C, 5 min isothermal, 12 °C/min, 30 min isothermal; FID 250 °C.

### Working Example 2: Oxidative carbonylation of 2,4-diaminotoluene (2,4-TDA) with methyl formate

A 160 mL Parr high pressure reactor was charged with methyl formate (30 mL, 0.49 mol), NaOCH₃ solution 25 wt% in methanol (0.115 ml, 0.5 mmol), supported Pd catalyst (0.011 mmol atom Pd) as listed in Table 2, and NaI (90 mg, 0.6 mmol). The reaction mixture was stirred at 700 rpm and heated to the desired temperature (80-160 °C). After the temperature had stabilized and the internal pressure (Pin) had become almost constant, the reactor was pressurized with artificial air (50% of Pin). Thereafter, 2,4-diaminotoluene (2,4-TDA) (0.61 g, 5 mmol) dissolved in methanol (5 mL) was injected into the reactor (4 mL/min) using a high pressure HPLC pump. The injection of 2,4-TDA was considered as start of the reaction (t = 0). After 4 h, the reaction was quenched by cooling the reactor with ice-water. The autoclave was opened and the reaction products were analyzed by GC as in example 1. > 99% conversion and 81% Yield for Toluene di Carbamate was achieved.

### Comparative Example 3: Oxidative carbonylation of aniline with CO/CH₃OH (conventional system)

A 160 mL Parr autoclave reactor was charged with MeOH (25 ml, 0.62 mmol), NaOCH₃ solution 25 wt% in methanol (0.115 ml, 0.5 mmol), aniline (0.93 g, 10 mmol), supported Pd catalyst (0.011 mmol atom Pd) as listed in Table 2, and NaI (90 mg, 0.6 mmol). The reaction mixture was stirred at 700 rpm and heated to 140 °C. After the temperature had stabilized, the reactor was pressurized first with CO and then artificial air to the desired pressure (P(air) was 50% of P(CO)). The pressurizing the reactor with air was considered as start of the reaction (t = 0). After 2 h, the reaction was quenched by cooling the reactor with ice-water. The autoclave was opened and the reaction products were analyzed by GC and GC-MS as in example 1.

The reactions described in working example 1 and comparative example 3 were carried out in two steps:

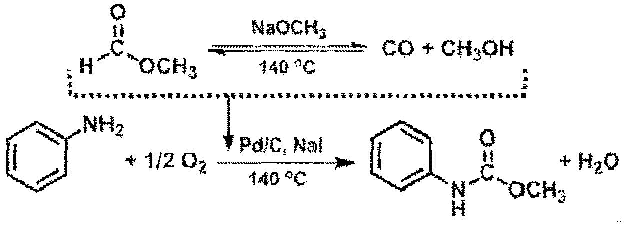

Step 1: Activation of methyl formate catalyzed by NaOCH₃. As mentioned above, the activation of methyl formate establishes the equilibrium between methyl formate and CO/CH₃OH. The formation of CO during the activation of methyl formate (depending on the amount of NaOCH₃) caused an increase of the internal pressure in the reactor to 30-80 bar. Thereafter, this internal pressure is called p_{CO}. It should be noted here that no external CO gas was added into the reactor.

Step 2: Oxidative carbonylation of aniline over Pd catalyst. After the equilibrium had been stabilized (constant temperature and p_{CO}), aniline and air were charged into the reactor to start the oxidative carbonylation reaction.

Using Pd supported on SiO₂, Al₂O₃ and C very similar results were obtained at 140°C (Table 2), whereby conversions of aniline higher than 95% and selectivities to MPC higher than 70% were achieved. Varying the Pd content in the carbon supported catalyst, the best performance was observed for Pd(10%)/C (entry 4). Prolonging this reaction up to 4 h, almost full conversion of aniline to MPC was achieved (entry 5).

**Table 2: The results of oxidative carbonylation of aniline by methyl formate using supported-Pd catalysts at 140 °C in 2 h.**

| **Entry** | **Catalyst** | **Conv.^{a} (%)** | **Sel.^{b} (%)** | **TOF^{c}** |
|---|---|---|---|---|
| 1 | Pd(5%)/SiO₂ | 95.2 | 72.8 | 300 |
| 2 | Pd(5%)/Al₂O₃ | 97.4 | 82.4 | 368 |
| 3 | Pd(5%)/C | 97.7 | 81.9 | 364 |
| 4 | Pd(10%)/C | 96.2 | 85.4 | 372 |
| 5 | Pd(10%)/C^{d} | 99.7 | 98.5 | 223 |
| 6 | Pd(1%)/C | 94.8 | 79.6 | 343 |
| 7 | Pd(1%)/C^{e} | 87.8 | 62.3 | 249 |

| | | | | |
|---|---|---|---|---|
| ^{a} conversion of aniline, ^{b} selectivity to MPC, ^{c} mmol MPC produced per mmol Pd per hour. ^{d} reaction time 4h, ^{e} catalyst recovered from entry 6 | | | | |

Comparing the results obtained with methyl formate of working example 1 to the corresponding conventional CO/CH₃OH system of comparative example 3 demonstrated advantages of the methyl formate route. Higher yields were achieved at lower reaction temperature as well as higher catalytic activity in terms of TOF. The supported-Pd catalysts showed good sustainability in this reaction (Table 2, entry 7). More than 70% of activity remained in the second run using the recycled catalyst, taking into account that Pd took part in a redox reaction in which there is high potential for Pd atoms to be voided from catalytic reaction via inactive Pd(0) species formation.

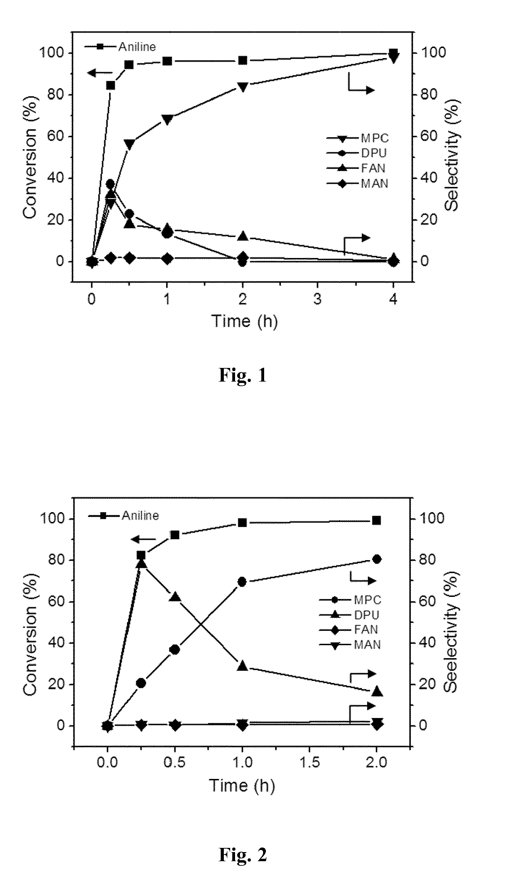

The oxidative carbonylation of aniline with methyl formate was performed to screen the product distribution in a wide temperature range of 80-160 °C in 2 h reaction (Figure 2). Apart from methyl phenylcarbamate (MPC), 1,3-diphenylurea (DPU), formanilide (FAN) and N-methylaniline (MAN) were identified in the reaction mixture. At 80 °C, less than 40% of aniline was converted and FAN was the main product. Increasing the temperature to 100 °C, the conversion of aniline increased to higher than 80% and the main product was DPU. DPU was also shown to be the main product of the oxidative carbonylation of aniline by CO in the temperature range of 100-120 °C. At 120 °C, the MPC formation was more pronounced and MPC was the main product. By performing the reaction at 140 °C, the highest selectivity to MPC was achieved (> 80%), while no DPU was detected after 2 h. It can be said that, here, the role of the main intermediate for MPC is shifted from DPU to FAN. The conventional route using CO/CH₃OH in the presence of methanol required temperatures up to 170 °C to achieve such a yield for MPC. Further increase in temperature to higher than 140 °C (up to 160 °C), did not significantly improve the selectivity to MPC. The later observation can be related to the decarbonylation of methyl formate equilibrium, which at temperatures higher than 140 °C does not shift further to the right.

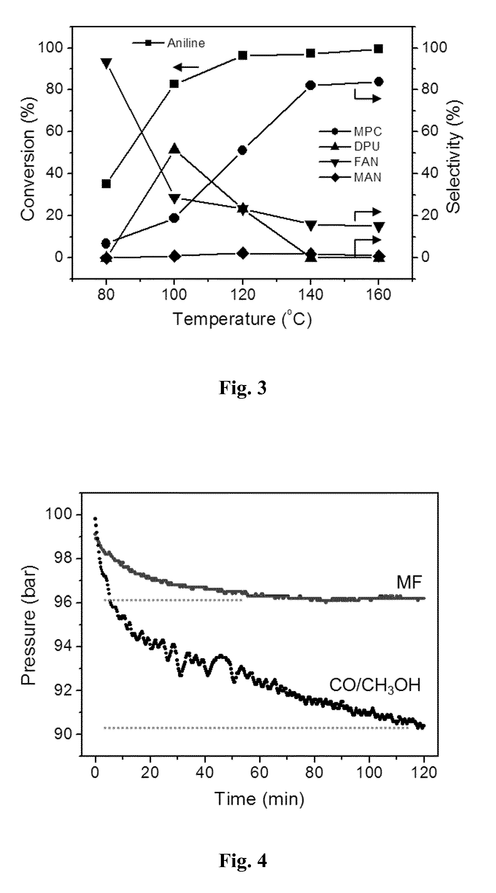

In order to monitor the reaction path of the oxidative carbonylation of aniline by methyl formate, the reactions were carried out at 140 °C in periods 0.25 to 4 h. The product distribution of these reactions is shown in Figure 3. In 0.25 h reaction, a very fast conversion of aniline was observed and MPC, DPU and FAN were produced with equal selectivities. In the reaction periods of 0.5 to 2 h, the selectivity to MPC always dominated, maintaining a progressive trend by time while DPU and FAN reduced apparently through the conversion to MPC. Meanwhile, it seems that diphenylurea has higher priority in conversion to MPC than formanilide.

### Comparative Example 4: Blank tests without catalyst and with methyl formate

A 160 mL Parr high pressure reactor was charged with methyl formate (30 mL, 0.49 moles), NaOCH₃ solution 25 wt% in methanol (0.115 ml, 0.5 mmol). The reaction mixture was stirred at 700 rpm and heated to 140 °C. After the temperature had stabilized and the internal pressure (Pin) had become almost constant, the reactor was pressurized with artificial air (50% of Pin). Immediately thereafter, aniline (0.93 g, 10 mmol) dissolved in methanol (5 mL, 0.12 moles) was injected into the reactor (4 mL/min) using a high pressure HPLC pump. The injection of aniline was considered as start of the reaction (t = 0). After 2 h, the reaction was quenched by cooling the reactor with ice-water. The autoclave was opened and the reaction products were analyzed by Gas Chromatography (GC) as in example 1.

### Comparative Example 5: Blank tests without catalyst and with CO/CH₃OH

A 160 mL Parr autoclave reactor was charged with MeOH (25 ml, 0.62 moles), NaOCH₃ solution 25 wt% in methanol (0.115 ml, 0.5 mmol), and aniline (0.93 g, 10 mmol). The reaction mixture was stirred at 700 rpm and heated to 140 °C. After the temperature had stabilized, the reactor was pressurized first with CO and then artificial air to the desired pressure (P(air) was 50% of P(CO)). The pressurizing the reactor with air was considered as start of the reaction (t = 0). After 2 h, the reaction was quenched by cooling the reactor with ice-water. The autoclave was opened and the reaction products were analyzed by GC as in example 1.

A reference reaction without Pd catalyst and the promoter (NaI) and in the presence of NaOCH₃ is described in comparative examples 4 and 5. Using CO/CH₃OH no conversion of aniline was obtained within 2 h, while the reaction with methyl formate provided 45% conversion of aniline and >97% selectivity to FAN. In other words, FAN was produced from methyl formate and not free CO.

In conclusion, the MPC formation from aniline using methyl formate follows two different parallel sequences: i) via the released CO/CH₃OH system through the DPU intermediate (examples 1 & 3) and ii) via FAN formation with methyl formate followed by catalytic reaction with methanol (examples 4 & 5). FAN as an intermediate for MPC unlike CO/CH₃OH route avoids the recycling of aniline which allows having higher yield for MPC at lower temperature.

The reactor pressure profiles during the reactions with methyl formate and CO/CH₃OH systems showed the CO and O₂ consumptions by the oxidative carbonylation (Figure 4). Since the final yields were comparable, the O₂ consumption for both systems could be assumed similar. Thus, the difference in the decrease of pressure in the reactor can be only due to the consumption of CO. For the CO/CH₃OH system, the CO consumption amount was more than two times higher compared to methyl formate. This observation supports the above conclusion that, in methyl formate route, the carbonylation was mainly supplied by methyl formate through formanilide formation than CO.

The efficiency of the oxidative carbonylation of aniline with methyl formate under different pressures of CO (P_{CO}), adjusted by the amount of NaOCH₃ was studied (Table 3). It was observed that to achieve high conversion of aniline and selectivity to MPC, P_{CO} higher than 30 bar is required. In addition, the presence of NaOCH₃ as strong base is essential to deprotonate CH₃OH and formanilide and push forward Pd catalyzed MPC formation.

**Table 3: The oxidative carbonylation of aniline by methyl formate in 2 h under different PCO adjusted by the amount of NaOCH₃ (0.15-0.5 mmol).**

| **Entry** | **P_{CO} (bar)** | **Aniline conv.¹/%** | **MPC sel.² / %** | **FA sel.² / %** | **MA sel.2 / %** |
|---|---|---|---|---|---|
| 1 | 12 | 50.4 | 6.7 | 66.7 | 26.5 |
| 2 | 38 | 96.4 | 74.8 | 22.2 | 3.1 |
| 3 | 47 | 97.2 | 75.2 | 22.5 | 2.8 |
| 4 | 67 | 96.2 | 85.4 | 14.1 | 1.6 |

| | | | | | |
|---|---|---|---|---|---|
| ¹ Conversion, ² Selectivity | | | | | |

In conclusion, MPC was synthesized from aniline and methyl formate as carbonylating agent in high yield and under relatively mild conditions (140 °C). The reaction does not require an external CO source. The product distribution and the analysis of the reaction profile suggest FAN as a key intermediate, which opens a new route to MPC formation. This provides new insight in an alternative mechanism for a reaction that is becoming more and more appealing for the greener production of isocyanates and other important chemicals. The method is expected to be applicable for other important amines and diamines used in polyurethane production.

## Claims

1. Method for producing alkylaryl carbamates, wherein alkyl formate and at least one amino aryl compound are reacted in presence of at least one metal-based catalyst.

2. Method for producing alkylaryl carbamates according to claim 1, wherein the alkyl formate is selected from the group comprising alkyl formates with a carbon chain from C1 to C6.

3. Method for producing alkylaryl carbamates according to one of the claims 1 or 2, wherein the at least one metal-based catalyst is a redox catalyst.

4. Method for producing alkylaryl caramates according to one of the claims 1 to 3, wherein the least one metal-based catalyst is on Palladium basis.

5. Method for producing alkylaryl carbamates according to one of the claims 1 to 4, wherein the catalyst is selected from the group consisting of PdBr₂, Pd(OAc)₂, Pd(salen) (salen: *N,N*'-bis(salicylidene)ethylenediamine), PdCl₂(CH₃CN)₂, Pd/C, Pd/SiO₂ and Pd/Al₂O₃.

6. Method for producing alkylaryl carbamates according to claim 5, wherein the Palladium concentration of the metal-based redox catalyst Pd/C, Pd/SiO₂ and Pd/Al₂O₃ is from 1 wt% to 20 wt%, preferably from 5 wt% to 10 wt%.

7. Method for producing alkylaryl carbamates according to claim 1, wherein further to the catalyst, a co-catalyst is used.

8. Method for producing alkylaryl carbamates according to claim 7, wherein the co-catalyst is on iodide basis.

9. Method for producing alkylaryl carbamates according to claim 8, wherein the co-catalyst is selected from the group consisting ofNaI, KI, LiI and CsI.

10. Method for producing alkylaryl carbamates according to one of the preceding claims, wherein the alkyl formate is activated by an alkali metal alkoxide MOCH₃.

11. Method for producing alkylaryl carbamates according to claim 10, wherein M is Na or K.

12. Method for producing alkylaryl carbamates according to one of the preceding claims, wherein the amino aryl compound is of the general formula (I) PhNH₂RR' where R and R' are each independently NH₂, H, alkyl, aryl or aminoaryl groups.

13. Method for producing alkylaryl carbamates according to one of the preceding claims, wherein the amino aryl compound is selected from the group comprising aniline, aminotoluene, 2,4-diaminotoluene (2,4-TDA) and 4,4'-methylenedianiline (4,4'-MDA), preferably 2,4-diaminotoluene (2,4-TDA) and 4,4'-methylenedianiline (4,4'-MDA), more preferably 2,4-diaminotoluene (2,4-TDA).

14. Method according to one of the preceding claims, wherein the reaction temperature is from 80 °C to 250 °C, preferably from 140 °C to 160 °C.

15. Method according to one of the preceding claims, wherein the reaction pressure is from 10 bar to 200 bar, preferably from 30 bar to 80 bar.
